# EUROPEAN PATENT APPLICATION

(11) **EP 1 634 553 A1**
(43) Date of publication of application: **15.03.2006**
(21) Application number: 05019818.3
(22) Date of filing: 12.09.2005
(51) Int. Cl.: A61F 5/01

(54) **Dorsal brace for ankle joint**

(30) Priority: 14.09.2004 IT BO20040564
(71) Applicant: Ortopedia A.S.O.R. Sanitari S.N.C., 40100 Bologna (IT)
(72) Inventor: Gasperini, Omer, 40100 Bologna (IT); Sanmartini, Gilberto, 40050 Monte San Pietro (Bo) (IT)
(74) Representative: Coppi, Cecilia

(57) **Abstract**

Dorsal brace for ankle joint wich comprises anatomically shaped plate means (1) for correcting the foot position intended to adhere on the upper side of the tarsal back, means (3a-3b) for positioning and supporting said plate means, and means 10 for movably fastening said positioning and supporting means.

## Description

The present invention relates to a dorsal brace for ankle joint.

Various foot pathologies are known and among these the equinovarus foot (from which the term equinus deformity derives) that can be flaccid foot or spastic foot can be mentioned. Equinus deformity is an abnormal position of the foot that rests only by the too much stretched front part. It is certainly the most frequent deformity and it is object of orthotic treatment to the lower limb of the patient. The plurality of causes require a proper estimating and diagnostic approach able to differentiate spasticity, muscle weakness, joint restriction upon which the deformity can be based. A position in equinus deformity leads to a considerable instability during one-foot load in stance phase that sometimes it is possible only on the outer edge of the forefoot; moreover it can make difficult to go on during the swing phase because of relevant limb elongation.

From what said above it is clear how the above mentioned foot pathologies considerably interfere with modes for using footwears. In fact, what the patient most frequently ask for to the orthotist is to be helped to achieve, by the orthosis, an independence as great as possible during walking.

Generally, patients go to the orthotist with medical prescriptions of orthosis well defined as regards characteristcs thereof. Even footwears play a considerable role since they stabilize and make easier the use of orthosis. Various podologic problems become worse or come from pressure exerted by unsuitable footwears. Footwears have to be enough comfortable in order to provide the proper space for fingers, above all if deformed ones. They have to be easy to wear with orthosis (also by the patient) and, for this reason, they must have a large opening towards the tip. They have to be light and guarantee a good transpiration.

In order to compensate and treat said pathologies various orthetic braces have been realized having the aim of compensate said foot pathologies.

Particularly it has been found that some of them had such overall dimensions to cover the distance between the knee and the foot sole. Such size is too big above all considering the fact that the patient itself would like a brace making his walking as natural as possible even from an aesthetic point of view. When testing the orthosis it will be examine the real efficacy and functionality of the supports. Short orthosis for heel and ankle are easy to wear. They do not involve difficulties when fitting, they are worn also with normal footwears; they are provided with various shapes and features more or less padded on malleoli (they are not always effective).

Ankle-foot orthosis are divided substantially in three types:
- prearranged
- custom-made
- on plaster cast- or computerized system-based Custom-made orthosis, in turn, are divided in various classes:

- with entire bottom (including also fingers, shell or crossed type)
- with short bottom (aligned to metatarsi, shell type)
- spiral one with long or short bottom, made of polypropylene or composite materials. The ankle joint angle is always of 90° and fastenings are made of velcro. Long bottom orthosis helps more the forefoot support, but they condition the knee functionality. They are suited for patient affected by drop foot. The ones with short bottom are less suitable for forefoot drop since they have a shorter lever.

It is necessary to pay attention when manufacturing such braces since, being rather stiff, they do not have to touch anyway malleoli: a little rubbing at medial level affects deambulation by inwardly rotating and supinating the foot.

Finally it is to be considered that plantar braces remove sensibility of foot-sole-ground contact, causing bearing patient to unsurly walk.

Prefabricated spiral carbon orthosis are very stiff on the leg portion, correct heel position by aligning the foot by the spiral effect and have a spring function, that is they store up energy for giving it back when walking. This orthosis is fit for persons without great foot and leg deformities, since they are not suited for large changes, but are quite difficult to wear.

Recently new custom-made spiral orthosis have been made allowing a good result in supinated feet. If the knee has big loads (heavy patients or knee deflections on frontal plane in varus) the brace has a very short life. In these cases cast orthosis or computerized systems are used.

Another type of known orthosis are elastic ankle supports with front dorsal padded flap and provided with traction straps made of rubber of the caoutchouc type intended to adjust the ankle flexion. However such braces has the drawback of being unsuitable in case of concomitant blood circulation problems of lower limbs and they exert an action for correcting the joint giving the function of accomplishing the flexion correction of ankle to adjustment of traction straps. They have also intolerance problems and are difficult to wear.

Another example of a known brace is made by means of a double plate made of plastic material surrounding the heel and laterally the ankle; since it is kept in place by an elastic band that by passing under the foot sole cross on the tarsal arch then wrapping and closing itself at the leg above the ankle. Such brace is suitable to compensate side instability of the ankle, but it presses too much the foot, compromising the motion and a good transpiration. Actually it can be seen that the region in contact with the plastic material of which the brace is made of extends from above the ankle to about half of the foot plantar base.

It is a technical task of the present invention to overcome above mentioned drawbacks and to provide a dorsal brace for ankle joint able to effectively correct the wrong position and pathologies the foot is affected of, as the consequence of a central or peripherical neurologic damage.

The principle at the base of the present finding is to provide a lever with fulcrum centered on the foot back, wherein a load is exerted to an end (ankle) and a reaction force to the other end (metatarsal level footwear) so that it is lifted. The fulcrum does not press on the foot back, since the lever acts with the foot up, so unloaded, and so it does not cause any painful pressures on the foot itself. Within such techincal task, the present invention aim is further to manufacture a smaller, light brace, easy to wear, with a long life and able not to affect the foot natural transpiration.

Within such technical task, another aim of the present invention is to accomplish the preceding task by manufacturing a brace easy to be positioned and to be properly fastened to the patient ankle and at the same time easy to be worn and compatible with other possible orthosis, such as arch supports, finger braces, elastic stockings and the like.

Within such technical task, another aim of the present invention is to accomplish the preceding task by a simple structure, quite easy to practically make it, with a sure use and effective operation, as well as with a quite low manufacturing cost.

This task and these aims are all achieved by the present dorsal brace for ankle joint characterized in that it comprises anatomically shaped plate means for correcting the foot position intended to adhere on the upper side of the tarsal back, means for positioning and supporting said plate means, and means for movably fastening said positioning and supporting means.

Further details will be particularly clear and evident from the detailled description of a preferred, not exclusive embodiment, of a dorsal brace for ankle joint according to the invention, shown as an indication, but not a limiting one, in annexed drawing tables, wherein:
- fig. 1 is a side view of the present invention object during use, inserted inside an high lacing footwear;
- fig. 2 is a perspective top view of the present invention;
- fig. 3 is a side view of the present invention with fastening means in the open condition;

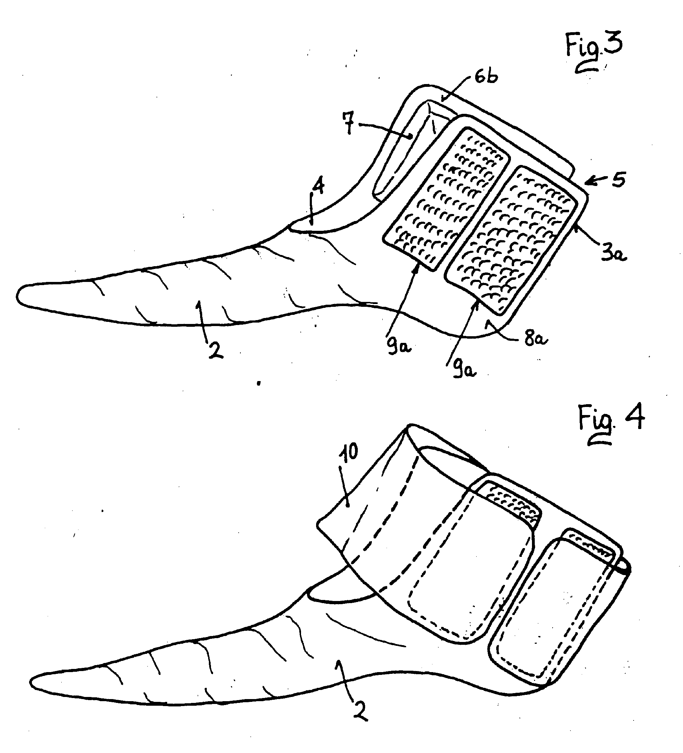

- fig. 4 is a side view of the present invention with fastening means in the closed condition.

Referring particularly to such figures the present dorsal brace for ankle joint according to the present invention is generally indicated by 1. The dorsal brace 1 is composed of an anatomically shaped plate 2 for correcting foot position composed of a semirigid plate made of a material of the thermoformable plastic type (e.g. polyethylene, polypropylene) or, alternatively, made of resin and fiber composite material.

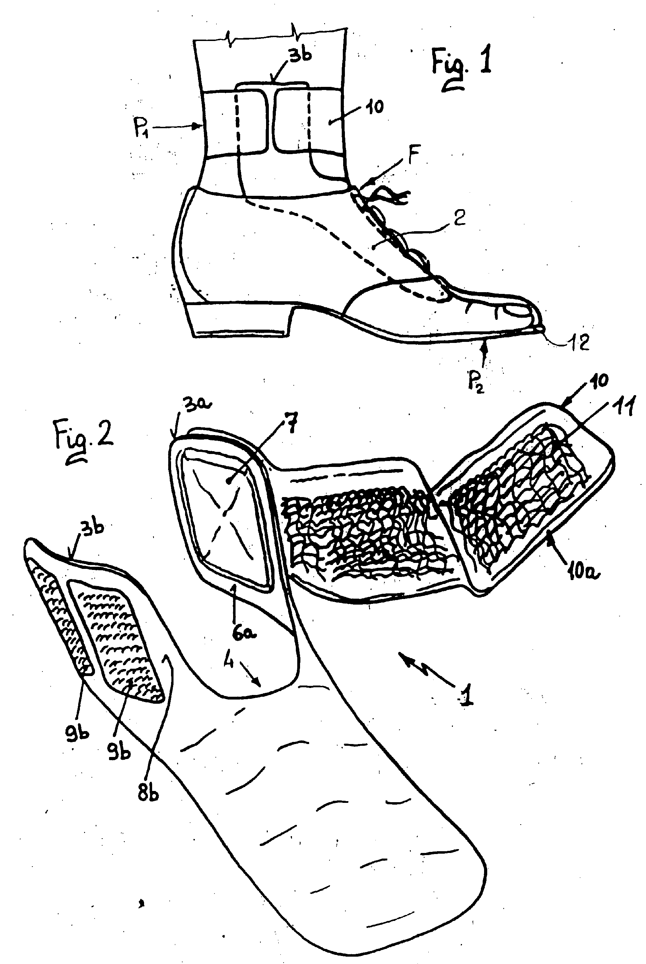

A pair of side ribs 3a, 3b (right and left) integral with the plate 2 project upwardly therefrom that is in the direction of the leg intended to adhere on the leg upon malleoli of ankles, such it can be better seen in fig. 1.

Side ribs 3a, 3b together with the shaped plate 2 determine a first front opening 4 and a second rear opening 5.

Said front and rear openings 4 and 5 allow the ankle joint to easily move as well as the brace 1 to be easily inserted upon socks and therefore inside the high or low laced footwear.

Both plate 2 and ribs 3a, 3b are monolithic since are made of the same material, and are manufactured such that the shape of the plate 2 can be easily adapted to the specific one of the foot whose position has to be corrected. Therefore each brace can be customized on individual patient.

On inner faces 6a and 6b of side ribs 3a and 3b respective anatomical pads 7 made of anallergic soft material are provided. By resting directly in contact with the leg, such pads 7 are intended to increase comfort of the present brace when it is worn.

On outer faces 8a and 8b of side ribs respective pairs of hooklike inserts 9a and 9b intended to make a movable coupling known as "velcro" type. Such movable coupling is realized by adhering on said hooklike inserts a corresponding stretch fabric strap 10 having on one face 10a a plurality of thin fibres 11 with a random chaotic pattern. Fibres 11 in contact with the corresponding plurality of small hooks on inserts 7a, 8a and 7b, 8b causes respective coupling and fastening.

The operation of the present invention is simple and intuitive and it can be summed up in the following.

The brace 1 can be grasped by the two side ribs 3a and 3b in order to be properly positioned with the plate 2 on the foot tarsal arch.

The foot have to be covered by a sock made of a fabric suitable for transpiration. Once positioned, malleoli of the ankle have to be controlled to be by pads 5 inside secured on side ribs, that in order to guarantee comfort and wearing ability of brace 1.

The foot and the brace 1 are inserted inside a low or high lacing footwear, such as the one that can be seen in fig. 1. Then the ankle is closed between side ribs 2 by means of the stretch strap 10 that is brought to adhere to hooklike inserts 9a and 9b with a coupling known as "velcro" type.

In order to re-open the brace it is sufficient to slightly draw an edge of the strap 10 in a direction perpendicular to the one in which it is in the fastened position. So by tearing it is possible to open the strap 10, to take off the footwear and so the whole brace 1.

The lever realized by the brace has fulcrum F at the foot back; so the pushing force P₁ exerted by the strap 10 at the ankle, causes an upwardly reaction P₂ on the footwear, in such a way that the foot tip is constrained to lift up under the action of the sole 12.

So it has been seen how the invention achieves proposed aims.

The invention expressed in this way is susceptible of changes and variants all falling within the scope of the inventive concept.

Moreover all details can be replaced by others that are technically equivalent.

Practically, used materials, as well as shapes and sizes, may be of any type depending on requirements without departing from the protection scope of following claims.

## Claims

1. Dorsal brace for ankle joint **characterized in that** it comprises anatomically shaped plate means (1) for correcting the foot position intended to adhere on the upper side of the tarsal back, means (3a-3b) for positioning and supporting said plate means, and means 10 for movably fastening said positioning and supporting means.

2. Dorsal brace for ankle joint, according to claim 1, **characterized in that** said anatomically shaped plate means for correcting the foot position comprise a semirigid plate (2) made of a preferably thermoformable material intended to be inserted inside a lacing footwear, upon the tarsal back and to cooperate with said footwear in order to keep the foot in a proper foot position.

3. Dorsal brace for ankle joint, according to one or more of the preceding claims, **characterized in that** means for positioning and supporting said plate means (2) comprise two side ribs (3a-3b) integral with said plate means (2), said ribs extending laterally to ankle malleoli by means of interposed anatomic pads (7).

4. Dorsal brace for ankle joint, according to one or more of the preceding claims, **characterized in that** means for movably fastening said positioning and supporting means comprise at least a pair of hooklike inserts (9a-9b) intended to make a movable coupling known as "velcro" type with a corresponding fabric strap 10 having a plurality of thin fibres 11 with a random chaotic pattern.

5. Dorsal brace for ankle joint, according to one or more of the preceding claims, **characterized in that** said semi-rigid plate (2) made of thermoformable material is composed of a material of the polyethylene and polypropylene type.

6. Dorsal brace for ankle joint, according to one or more of the preceding claims, **characterized in that** said anatomical pads (7) are made of a soft material and intended to anatomically adhere on the leg surface upon ankle malleoli.

7. Dorsal brace for ankle joint, according to one or more of the preceding claims, **characterized in that** said pads (7) are secured to respective inner faces of said side ribs by gluing.

8. Dorsal brace for ankle joint, according to one or more of the preceding claims, **characterized in that** said hooklike inserts (9a-9b) are secured on respective outer faces 8a-8b of said side ribs by gluing.

9. Dorsal brace for ankle joint, according to one or more of the preceding claims, **characterized in that** said fabric strap (10) having a plurality of thin fibres with a random chaotic pattern is of the semi-elastic type.

10. Dorsal brace for ankle joint, according to one or more of the preceding claims, **characterized in that** said anatomical pads made of soft material are of the anallergic type.

11. Dorsal brace for ankle joint, according to one or more of the preceding claims, and according to what disclosed and shown for specified aims.
